# EUROPÄISCHE PATENTANMELDUNG

(11) **EP 2 074 940 A1**
(43) Veröffentlichungstag der Anmeldung: **01.07.2009**
(21) Anmeldenummer: 08075938.4
(22) Anmeldetag: 11.12.2008
(51) Int. Cl.: A61B 5/00

(54) **Zahnbehandlungs- oder -untersuchungsvorrichtung**

(30) Priorität: 18.12.2007 DE 102007063419
(71) Anmelder: Carl Zeiss Surgical GmbH, 73447 Oberkochen (DE)
(72) Erfinder: Hauger, Christoph, 73431 Aalen (DE)
(74) Vertreter: Theobald, Andreas

(57) **Zusammenfassung**

Es wird eine Zahnbehandlungs- oder -untersuchungsvorrichtung zur Verfügung gestellt mit:
- einem Weißlichtinterferometer (7), welches einen Messstrahlzweig (19), einen Referenzstrahlzweig (17), einen Emitter (31) zum Emittieren von Messstrahlung in Richtung auf einen zu behandelnden bzw. zu untersuchenden Zahn, einen Empfänger (31) zum Empfang von Messstrahlung, welche vom zu behandelnden bzw. zu untersuchenden Zahn reflektiert worden ist, einen Überlagerer (21) zum Überlagern von Referenzstrahlung und reflektierter Messstrahlung und eine Detektor (23) zum Detektieren der überlagerten Strahlung und zum Erzeugen eines die überlagerte Strahlung repräsentierenden Interferometriesignals umfasst,
- einem Zahnbehandlungs- oder -untersuchungsinstrument (1) mit einem distalen Ende (5), in dem der Emitter (31) und der Empfänger (31) angeordnet sind und das derart ausgestaltet ist, dass es an den zu behandelnden bzw. zu untersuchenden Zahn eines Patienten herangeführt werden kann,
- einer dem Weißlichtinterferometer (7) zugeordneten Steuereinheit (13) mit einem Steuerprogramm, welches das Weißlichtinterferometer (7) dazu veranlasst, Tiefenscans vom Behandlungs- bzw. Untersuchungsobjekt durchzuführen,
- einer mit dem Detektor (23) zum Empfang des Interferometriesignals verbundenen Analyseeinrichtung (9), die zum Ermitteln des Abstands der Pulpa des zu behandelnden bzw. zu untersuchenden Zahns von dessen aktueller Oberfläche und zur Ausgabe eines den Abstand repräsentierenden Abstandssignals ausgebildet ist, und
- einer mit der Analyseeinrichtung (9) zum Empfang des Abstands verbundenen Warn- und/oder Stoppeinrichtung (11), die zum Ausgeben eines Warnsignals und/oder zum Anhalten des Zahnbehandlungs- oder - untersuchungsinstruments (1), wenn ein vorbestimmter Mindestabstand erreicht oder unterschritten ist, ausgestaltet ist.

## Beschreibung

Die vorliegende Erfindung betrifft eine Zahnbehandlungs- oder -untersuchungsvorrichtung mit einem Zahnbehandlungs- oder -untersuchungsinstrument. Daneben betrifft die vorliegende Erfindung ein Verfahren zum Steuern eines Zahnbehandlungs- oder -untersuchungsinstrumentes sowie ein Verfahren zum Überwachen eines Zahnbehandlungs- oder -untersuchungsinstrumentes.

Ein Zahn besteht im Wesentlichen aus drei Komponenten, nämlich dem Dentin, auch Zahnbein genannt, dem Zahnschmelz, mit dem das Dentin zu dessen Schutz überzogen ist, sowie der Pulpa, auch Zahnmark oder umgangssprachlich Zahnnerv genannt, welche die Nervenfasern und Blutgefäße des Zahnes enthält. Die Pulpa lässt sich unterteilen in die Kronenpulpa, die durch die Zahnkrone verläuft, und die Wurzelpulpa, die durch die Wurzel verläuft. Das Pulpagewebe ist außerordentlich empfindlich, beispielsweise gegenüber thermischen Reizen. Temperaturen über 42° C können das Pulpagewebe thermisch schädigen, sodass es zum Absterben von Pulpagewebe kommen kann.

Für das Präparieren von Zähnen ist die Kenntnis der Größe und der Ausdehnung der Kronenpulpa von Interesse, um ein unbeabsichtigtes Öffnen der Kronenpulpa beim Präparieren eines Zahnes zu vermeiden. Zum Ermitteln der Pulpaausdehnung kommen oft radiologische Darstellungsmethoden zur Anwendung. In radiologischen Darstellungen, wie beispielsweise bei Bissflügelaufnahmen, werden die tatsächlichen Abmessungen der Pulpa häufig aber nicht korrekt wiedergegeben. Insbesondere im Bereich der hinteren Backenzähne (Molaren) kann es zum Unterschätzen der Pulpaausdehnung kommen.

Aus der DE 10 2005 044889 A1 ist ein zahnmedizinisches Untersuchungs- und/oder Behandlungswerkzeug bekannt, welches ein Lumen aufweist, in dem ein Bildgebungskatheter zur Aufnahme von Bilddaten eines Untersuchungs- und/oder Behandlungsbereiches angeordnet ist. Der Bildgebungskatheter wird zur Aufnahme von Bilddaten nach einer teilweisen Pulpaentfernung im Wurzelkanal herangezogen. Insbesondere kann der Bildgebungskatheter ein OCT-Katheter sein, also ein Katheter zum Durchführen einer optischen Kohärenztomografie.

Aus Patent Abstracts of Japan 2004344260A ist ein zahnmedizinisches Diagnosegerät bekannt, welches eine optische Kamera sowie ein OCT-Gerät aufweist. Mit der optischen Kamera wird ein Oberflächenbild eines Zahnbereiches aufgenommen, in welchem dann ein Scan mit dem OCT-Gerät aufgenommen wird.

Sowohl in DE 10 2005 044 889 A1 als auch in Patent Abstracts of Japan geht es nicht darum, ein unbeabsichtigtes Öffnen der Pulpa zu vermeiden. Insbesondere ist in DE 10 2005 044 889A1 gerade das Öffnen der Pulpa beabsichtigt.

Es besteht daher ein Bedarf an Zahnbehandlungs- und -untersuchungsvorrichtungen, die ein unbeabsichtigtes Öffnen der Pulpa vermeiden helfen. Weiterhin besteht ein Bedarf an Verfahren zum Steuern und Überwachen derartiger Geräte, die dazu beitragen, das unbeabsichtigte Öffnen der Zahnpulpa zu vermeiden.

Eine erste Aufgabe der vorliegenden Erfindung ist es daher, eine Zahnbehandlungs- oder -untersuchungsvorrichtung zur Verfügung zu stellen, welche das unbeabsichtigte Öffnen der Zahnpulpa vermeiden hilft. Es ist eine zweite Aufgabe der vorliegenden Erfindung, ein Verfahren zum Steuern eines Zahnbehandlungs- oder -untersuchungsinstrumentes zur Verfügung zu stellen, welches dazu beiträgt, das unbeabsichtigte Öffnen der Zahnpulpa zu vermeiden. Eine dritte Aufgabe der vorliegenden Erfindung ist es, ein Verfahren zum Überwachen eines Zahnbehandlungs- oder -untersuchungsinstruments zur Verfügung zu stellen, welches dazu beiträgt, ein unbeabsichtigtes Öffnen der Zahnpulpa zu vermeiden.

Die erste Aufgabe wird durch eine Zahnbehandlungs- oder -untersuchungsvorrichtung nach Anspruch 1 gelöst, die zweite Aufgabe durch ein Verfahren zum Steuern eines Zahnbehandlungs- oder -untersuchungsinstruments nach Anspruch 9 und die dritte Aufgabe durch ein Verfahren zum Überwachen eines Zahnbehandlungs- oder -untersuchungsinstruments nach Anspruch 10. Die abhängigen Ansprüche enthalten vorteilhafte Ausgestaltungen der Erfindung.

Eine erfindungsgemäße Zahnbehandlungs- oder -untersuchungsvorrichtung umfasst ein Zahnbehandlungs- oder -untersuchungsinstrument, ein Weißlichtinterferometer, eine Steuereinheit, eine Analyseeinrichtung und eine Warn- und/oder Stoppeinrichtung, die zum Ausgeben eines Warnsignals und/oder zum Anhalten des Zahnbehandlungs- oder -untersuchungsinstrumentes ausgestaltet ist. Das Weißlichtinterferometer weist einen Messstrahlzweig, einen Referenzstrahlzweig, einen Emitter zum Emittieren von Messstrahlung in Richtung auf einen zu behandelnden beziehungsweise zu untersuchenden Zahn, einen Empfänger zum Empfangen von Messstrahlung, welche vom zu behandelnden beziehungsweise zu untersuchenden Zahn reflektiert worden ist, einen Überlagerer zum Überlagern von Referenzstrahlung und reflektierter Messstrahlung sowie einen Detektor zum Detektieren der überlagerten Strahlung und zum Erzeugen eines die überlagerte Strahlung repräsentierenden Interferometriesignals auf. Das Zahnbehandlungs- oder Untersuchungsinstrument weist ein distales Ende auf, in dem der Emitter und der Empfänger angeordnet sind. Es ist derart ausgestaltet, dass es an den zu behandelnden beziehungsweise zu untersuchenden Zahn eines Patienten herangeführt werden kann. Insbesondere können der Emitter und der Empfänger im Zahnbehandlungs- oder Untersuchungsinstrument identisch sein. Die Steuereinheit ist dem Weißlichtinterferometer zugeordnet und umfasst ein Steuerprogramm, welches das Weißlichtinterferometer dazu veranlasst, Tiefenscans vom Behandlungs- beziehungsweise Untersuchungsobjekt durchzuführen. Derartige Tiefenscans können durch das Steuerprogramm entweder kontinuierlich veranlasst werden oder lediglich bei Vorliegen eines Auslösesignals. Die Tiefenscans werden gelegentlich auch A-Scans genannt. Die Analyseeinrichtung ist mit dem Detektor des Weißlichtinterferometers zum Empfang des Interferometriesignals verbunden. Sie ist zum Ermitteln des Abstandes der Pulpa des zu behandelnden beziehungsweise zu untersuchenden Zahnes von dessen aktueller Oberfläche und zur Ausgabe eines den Abstand repräsentierenden Abstandssignals ausgebildet. Die aktuelle Oberfläche des Zahns kann entweder die eigentliche Zahnoberfläche oder die Oberfläche einer Zahnöffnung, wie beispielsweise einer Bohrung sein. Die Warn- und/oder Stoppeinrichtung ist mit der Analyseeinrichtung zum Empfang des Abstandsignals verbunden und zum Ausgeben eines Warnsignals und/oder zum Anhalten des Zahnbehandlungs- oder -untersuchungsinstrumentes, wenn ein vorbestimmter Mindestabstand erreicht oder unterschritten ist, ausgestaltet. Die erfindungsgemäße Zahnbehandlungs- oder -untersuchungsvorrichtung kann insbesondere auch als eine Untersuchungsvorrichtung ausgestaltet sein, die ausschließlich eine weißlichtinterferometrische Sonde als Zahnuntersuchungsinstrument enthält.

Die erfindungsgemäße Vorrichtung ermöglicht es, mithilfe von Weißlichtinterferometrie einen Tiefenbereich im Zahn zu untersuchen, ohne dass der Zahn dazu geöffnet werden müsste. Insbesondere lässt sich mittels des Tiefenscans die Grenze zwischen dem Dentin und der weicheren Pulpa sowie der Abstand dieser Grenze von der aktuellen Oberfläche leicht feststellen. Hierzu genügt in der Regel ein Tiefenscan im Bereich von 1 bis 3 mm Tiefenausdehnung. Es kann also mittels einer äußerlichen Untersuchung die Tiefe der Pulpa unter der Zahnoberfläche beziehungsweise unter der Fläche einer Zahnöffnung ermittelt werden. Wenn hierbei festgestellt wird, dass ein vorgegebener Mindestabstand zwischen der Oberfläche und der Pulpa erreicht ist, wird ein Warnsignal abgegeben, welches das Zahnbehandlungspersonal auf das Erreichen des vorgegebenen Mindestabstands zwischen der Oberfläche und der Pulpa aufmerksam macht. Wenn der Emitter und der Detektor des Weißlichtinterferometers in eine zahnmedizinische Behandlungsvorrichtung, beispielsweise einen zahnmedizinischen Bohrer integriert ist, besteht zudem die Möglichkeit, die Behandlungsvorrichtung anzuhalten, wenn der Mindestabstand erreicht ist. Im Falle des Bohrers würde dies bedeuten, dass der Bohrer abgeschaltet wird, sobald der Mindestabstand erreicht oder unterschritten ist. Durch das Warnsignal beziehungsweise das Abschalten des Behandlungsinstruments kann ein unbeabsichtigtes Öffnen der Pulpa weitgehend vermieden werden. Zudem besteht die Möglichkeit, dass das Behandlungspersonal bei Annäherung an die Pulpa nach dem Warnsignal die Behandlung mit erhöhter Sorgfalt durchführt, um das unbeabsichtigte Öffnen der Pulpa auch für den Fall zu vermeiden, dass der Mindestabstand aus medizinischen Gründen unterschritten werden muss.

In der erfindungsgemäßen Zahnbehandlungs- oder -untersuchungsvorrichtung kann der Messstrahlzweig insbesondere eine optische Faser mit einem distalen Ende umfassen, welches im distalen Ende des Zahnbehandlungs- oder -untersuchungsinstruments angeordnet ist. Emitter und Empfänger können hierbei durch das distale Faserende gebildet sein. Auf diese Weise können sehr kleine Abmessungen des Messstrahlzweiges im Bereich des Zahnbehandlungs- oder -untersuchungsinstruments realisiert werden, sodass der Emitter und der Detektor sehr nah an die Oberfläche des Zahns und insbesondere sehr nahe an die Oberfläche auch einer kleinen Bohrung im Zahn herangeführt werden kann. Außerdem ermöglichen kleine Faserdurchmesser die Beleuchtung eines lateral sehr kleinen Oberflächenbereiches, wodurch sich eine sehr hohe laterale Auflösung beim Tiefenscan erreichen lässt, was insbesondere vorteilhaft ist, wenn auch ein laterales Scannen erfolgen soll, um den lateralen Verlauf der Grenze zwischen Pulpa und Dentin zu ermitteln.

Als Zahnbehandlungsinstrument kann insbesondere ein Bohrer vorhanden sein, der ein Bohrwerkzeug mit einem distalen Borhwerkzeugende aufweist, in dem oder in dessen Nähe das distale Faserende angeordnet ist. Insbesondere beim Präparieren eines Zahnes mittels eines Bohrers besteht das Risiko einer unbeabsichtigten Öffnung der Pulpa. Die erfindungsgemäße Vorrichtung kann daher besonders vorteilhaft in einem Zahnbehandlungsinstrument mit Bohrer zum Vermeiden des unbeabsichtigten Öffnens der Pulpa zur Anwendung kommen. Die optische Faser kann hierbei insbesondere in der Rotationsachse des Bohrwerkzeugs des Bohrers angeordnet sein, sodass in einfacher Weise immer das Zentrum der aktuellen Oberfläche der Bohrung mit dem Tiefenscan vermessen wird.

Alternativ besteht die Möglichkeit, die optische Faser außerhalb des Bohrwerkzeugs so anzuordnen, dass das distale Faserende denjenigen Ort beleuchtet, an dem sich das distale Bohrwerkzeugende befindet. Das Anordnen des Faserendes in einem rotierenden Bohrwerkzeug ist dann nicht notwendig.

Die Steuereinheit der erfindungsgemäßen Zahnbehandlungs- oder -untersuchungsvorrichtung kann insbesondere mit einem Steuerprogramm versehen sein, welches das Weißlichtinterferometer dazu veranlasst, kontinuierlich Tiefenscans vom Behandlungs- beziehungsweise Untersuchungsobjekt durchzuführen. Auf diese Weise ist eine kontinuierliche Überwachung des Abstands der Oberfläche von der Pulpa gewährleistet. Es kann dann beim Erreichen des Mindestabstandes sofort das Warnsignal beziehungsweise das Stoppsignal ausgegeben werden. Als Alternative besteht die Möglichkeit, dass die Zahnbehandlungs- oder -untersuchungsvorrichtung ein vom Behandlungs- beziehungsweise Untersuchungspersonal zu bedienendes Auslöseelement umfasst. Dieses ist dann mit der Steuereinheit zur Abgabe eines den Tiefenscan auslösenden Auslösesignals verbunden. Das Auslöseelement kann beispielsweise eine Taste am Handstück, ein Fußschalter, et cetera sein. In dieser Ausgestaltung wird ein Tiefenscan nur bei Bedarf durchgeführt. Diese Ausgestaltung ist vor allem dann sinnvoll, wenn die Zahnbehandlungs- und -untersuchungsvorrichtung lediglich eine weißlichtinterferometrische Sonde als einziges Untersuchungsinstrument aufweist.

Um einen lateralen Verlauf der Grenze zwischen der Pulpa und dem Dentin ermitteln zu können, kann der Messstrahlzweig des Weißlichtinterferometers eine Scanvorrichtung aufweisen, mit der sich der mit der Messstrahlung bestrahlte Oberflächenbereich auf dem zu behandelnden beziehungsweise zu untersuchenden Zahn lateral versetzen lässt. Somit kann ein lateraler Scan (sogenannter B-Scan) vorgenommen werden, der dazu führt, dass an mehreren Punkten eines lateralen Bereiches jeweils ein Tiefenscan durchgeführt wird. Auf diese Weise lässt sich der Verlauf der Pulpa über einen größeren Oberflächenbereich ermitteln. Es besteht dann insbesondere die Möglichkeit, das Warnsignal oder das Stoppsignal auch dann auszugeben, wenn der Mindestabstand in einem Bereich unterschritten wird, der zwar nicht dem aktuellen Behandlungsbereich entspricht, aber in dessen unmittelbarer Nähe liegt.

In einem ersten erfindungsgemäßen Verfahren zum Steuern eines Zahnbehandlungsinstrumentes wird während der Zahnbehandlung mittels eines Weißlichtinterferometrieverfahrens ein Tiefenscan des behandelten beziehungsweise untersuchten Zahnbereiches vorgenommen. Aus dem Tiefenscan wird dann der Abstand der Oberfläche des behandelten Zahnbereiches von der Pulpa des Zahnes ermittelt. Das Zahnbehandlungsinstrument wird angehalten, wenn ein bestimmter Mindestabstand erreicht oder unterschritten wird. Auf diese Weise kann mittels weißlichtinterferometrischer Messung des Abstands der Pulpa von der Oberfläche ein unbeabsichtigtes Öffnen der Pulpa durch rechtzeitiges Anhalten des Behandlungsinstruments vermieden werden.

In einem zweiten erfindungsgemäßen Verfahren erfolgt ein Überwachen eines Zahnbehandlungs- oder -untersuchungsinstrumentes, indem während der Zahnbehandlung beziehungsweise der Zahnuntersuchung mittels eines Weißlichtinterferometrieverfahrens ein Tiefenscan des behandelten beziehungsweise untersuchten Zahnbereiches vorgenommen wird. Aus dem Tiefenscan wird dann der Abstand der Oberfläche des behandelten beziehungsweise untersuchten Zahnbereiches von der Pulpa des Zahnes ermittelt. Wenn ein bestimmter Mindestabstand erreicht oder unterschritten wird, wird ein Warnsignal abgegeben. Das Warnsignal kann hierbei das Behandlungspersonal dazu veranlassen, die Behandlung beziehungsweise Untersuchung zu beenden oder, falls dies noch nicht möglich ist, mit erhöhter Sorgfalt weiterzuführen.

Um auch den Abstand der Pulpa von der Oberfläche in Bereichen ermitteln zu können, die dem aktuellen Behandlungs- beziehungsweise Untersuchungsbereich benachbart sind, kann in beiden Verfahren zusätzlich zum Tiefenscan ein laterales Scannen des behandelten beziehungsweise untersuchten Zahnbereiches erfolgen. An jedem lateralen Punkt kann dann ein Tiefenscan vorgenommen werden.

Unabhängig davon, ob ein laterales Scannen erfolgt oder nicht, kann ein Tiefenscan kontinuierlich erfolgen oder nur nach Betätigen einer Auslösevorrichtung, beispielsweise einer Taste am Handstück, einem Fußschalter, et cetera, durch das Behandlungs- beziehungsweise Untersuchungspersonal. Das kontinuierliche Durchführen von Tiefenscans bietet sich insbesondere dort an, wo bei Erreichen des Mindestabstandes sofort zu reagieren ist. Falls das Erreichen des Mindestabstandes keine kritische Grenze markiert, sondern lediglich zur Information des Behandlungs- beziehungsweise Untersuchungspersonals dienen soll, kann es ausreichend sein, wenn ein Tiefenscan nur gelegentlich auf Veranlassung des Personals durchgeführt wird.

In beiden erfindungsgemäßen Verfahren können die Tiefenscans von der Oberfläche ausgehend bis in eine Tiefe von 1 bis 3 mm durchgeführt werden. Diese Tiefe ist in der Regel ausreichend, um das unbeabsichtigte Öffnen der Pulpa zu vermeiden.

Weitere Merkmale, Eigenschaften und Vorteile der vorliegenden Erfindung ergeben sich aus der nachfolgenden Beschreibung von Ausführungsbeispielen unter Bezugnahme auf die beiliegenden Figuren.
- Figur 1: zeigt schematisch eine Zahnbehandlungs- oder -untersuchungsvorrichtung in Form eines Blockschaltbilds.
- Figur 2: zeigt eine Ausführungsform der Vorrichtung aus Figur 1, in der ein Emitter und ein Empfänger in einen zahnmedizinischen Bohrer integriert sind.
- Figur 3: zeigt eine Abwandlung der Ausführungsform aus Figur 2.
- Figur 4: zeigt eine weitere Abwandlung der Ausführungsform aus Figur 1.
- Figur 5: zeigt eine Ausführungsform der Vorrichtung aus Figur 1, in der der Emitter und der Empfänger in eine zahnmedizinische Untersuchungssonde integriert ist.

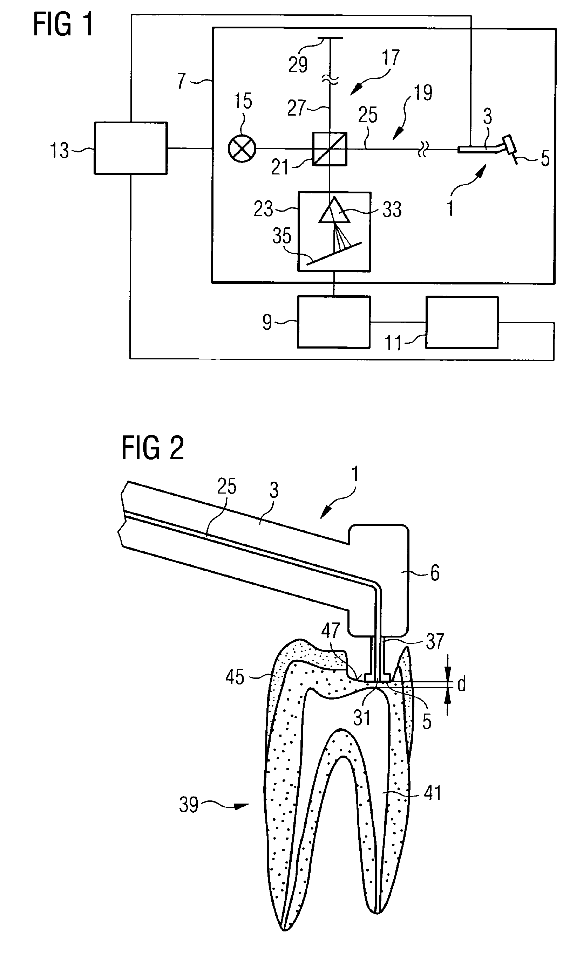

Eine erfindungsgemäße Zahnbehandlungs- oder -untersuchungsvorrichtung wird nachfolgend mit Bezug auf Figur 1 beschrieben. Die Vorrichtung umfasst ein Zahnbehandlungs- oder -untersuchungsinstrument 1, das ein Handstück 3 und ein distales Ende 5 aufweist und im vorliegenden Ausführungsbeispiel als zahnmedizinischer Bohrer dargestellt ist. Sie umfasst weiterhin ein Weißlichtinterferometer 7, eine Analyseeinrichtung 9, eine Warn- oder Stoppeinrichtung 11 sowie eine Steuereinheit 13. Die Analyseeinrichtung 9 und die Steuereinheit 13 sind mit dem Weißlichtinterferometer zum Empfang des Interferometriesignals beziehungsweise zur Abgabe von Steuersignalen verbunden.

Das Weißlichtinterferometer umfasst eine breitbandige Lichtquelle 15, welche eine kurze bis sehr kurze Kohärenzlänge aufweist. Das von der Lichtquelle ausgesandte Licht wird im Folgenden als Weißlicht bezeichnet, auch wenn das breitbandige Spektrum des Lichtes nicht unbedingt weißes Licht zu ergeben braucht. Von Bedeutung ist lediglich, dass die Lichtquelle breitbandig ist, da die zeitliche Kohärenz des Lichtes mit zunehmender Bandbreite geringer wird. Das Weißlichtinterferometer 7 umfasst außerdem einen Referenzstrahlzweig 17 und einen Messstrahlzweig 19, einen Aufteiler und Überlagerer 21, der im vorliegenden Ausführungsbeispiel als Strahlteiler dargestellt ist, und einen Detektor 23.

Im Weißlichtinterferometer 7 wird das von der breitbandigen Lichtquelle 15 ausgesendete Weißlicht vom Strahlteiler 21 in einen Messstrahl und einen Referenzstrahl aufgeteilt, die dann in den Referenzstrahlzweig 17 beziehungsweise den Messstrahlzweig 19 eingekoppelt werden. Die beiden Zweige umfassen jeweils eine Lichtleitfaser 25 beziehungsweise 27, in die das Referenzlicht beziehungsweise das Messlicht eingekoppelt werden. Im Referenzzweig wird das Messlicht zu einem Reflektor 29 geleitet und von dort durch die Lichtleitfaser 27 zurück zum Strahlteiler 21 geleitet. In ähnlicher Weise wird das Messlicht durch die Lichtleitfaser 25 zum distalen Ende 5 des Zahnbehandlungs- oder -untersuchungsinstrumentes 1 geleitet, wo es aus dem distalen Faserende 31 (in Figur 1 nicht dargestellt, vergleiche Figur 2) austritt. Messstrahlung wird dann im Zahn von der Grenzfläche zwischen der Pulpa und dem Dentin reflektiert, und ein Teil der reflektierten Messstrahlung gelangt durch das distale Faserende 31 wieder in die optische Faser 25 des Messstrahlzweiges 19. Das distale Faserende 31 dient also gleichzeitig als Emitter und als Empfänger von Messstrahlung beziehungsweise reflektierter Messstrahlung. Durch die optische Faser wird das reflektierte Messlicht zum Strahlteiler 21 geleitet. Dort wird das Messlicht dem Referenzlicht überlagert und die Überlagerung aus Messlicht und Referenzlicht dem Detektor 23 zugeleitet. Dieser enthält im vorliegenden Ausführungsbeispiel ein Spektroskop 33, das in der Figur als Prisma dargestellt ist, sowie eine Detektorzeile 35, die das vom Spektroskop 33 erzeugte Spektrum detektiert und in ein das Spektrum repräsentierendes Interferometriesignal umwandelt.

Damit in der Überlagerung aus Messlicht und Referenzlicht bei der vorhandenen kurzen Kohärenzlänge überhaupt Interferenzerscheinungen auftreten können, sind die Längen des Referenzzweiges 17 und des Messzweiges 19 derart aufeinander abgestimmt, dass der Wegunterschied zwischen dem Messlicht und dem Referenzlicht kleiner als die Kohärenzlänge des Weißlichtes ist. Ein Intensitätsmaximum kann für eine bestimmte Wellenlänge nur dann auftreten, wenn die vom Referenzlicht und vom Messlicht zurückgelegte Wegstrecke gleich ist. Mit zunehmenden Unterschied in der zurückgelegten Wegstrecke nimmt die Intensität ab. Aus der spektralen Intensitätsverteilung kann daher auf die Tiefe geschlossen werden, in der die Reflektion stattgefunden hat.

Obwohl im vorliegenden Beispiel die Tiefe, in der die Reflektion stattfindet, aus der spektralen Verteilung des überlagerten Lichtes ermittelt wird, ist es auch möglich, die Tiefe ohne spektrale Aufspaltung des überlagerten Lichtes zu ermitteln. In diesem Fall würde eine Vorrichtung vorhanden sein, mit der sich der Referenzstrahlzweig oder der Messstrahlzweig in der Länge justieren lassen. Aus der Justagestellung, bei der im überlagerten Licht ein Maximum auftritt, lässt sich dann ebenfalls auf die Tiefe schließen, in der die Reflektion stattfindet.

Konkrete Ausgestaltungen für Weißlichtinterferometer sind beispielsweise in DE 199 29 406 A1 oder in EP 1 314 953 A2 beschrieben. Auf diese Dokumente wird hinsichtlich einer möglichen konkreten Ausgestaltung für das Weißlichtinterferometer der erfindungsgemäßen Zahnbehandlungs- oder -untersuchungsvorrichtung Bezug genommen.

Vom Detektor 23 wird ein Interferometriesignal ausgegeben, das im vorliegenden Beispiel die spektrale Intensitätsverteilung der Überlagerung aus Messlicht und Referenzlicht repräsentiert. Dieses Interferenzsignal wird von der Analyseeinrichtung 9, die mit dem Weißlichtinterferometer zum Empfang des Interferometriesignals verbunden ist, empfangen und ausgewertet. Im vorliegenden Fall beinhaltet die Auswertung im Wesentlichen eine Fourier-Transformation der spektralen Intensitätsverteilung, aus der sich dann die Tiefe, in der die Reflektion stattgefunden hat, ermitteln lässt. Die ermittelte Tiefe wird von der Analyseeinrichtung als Abstandssignal ausgegeben, welches den Abstand der Pulpa von der Oberfläche, welcher das Messlicht zugeführt worden ist, repräsentiert. Das Abstandssignal wird von der mit der Analyseeinrichtung 9 zum Empfang des Abstandssignals verbundenen Warn- oder Stoppeinrichtung mit einem vorgegebenen Mindestabstand, der in einem Speicher der Warn- und/oder Stoppeinrichtung gespeichert ist, verglichen. Wenn der Vergleich ergibt, dass der Mindestabstand erreicht oder unterschritten ist, gibt die Warn- und/oder Stoppeinrichtung ein akustisches oder optisches Warnsignal aus. Zusätzlich oder alternativ kann sie auch auf das Untersuchungs- oder Behandlungsinstrument 1 einwirken, um die Untersuchung beziehungsweise Behandlung anzuhalten, beispielsweise einen Bohrer anzuhalten.

Gesteuert wird die Zahnbehandlungs- oder -untersuchungsvorrichtung von der dem Weißlichtinterferometer zugeordneten Steuereinheit 13, die ein Steuerprogramm enthält, welches das Weißlichtinterferometer dazu veranlasst, kontinuierlich Tiefenscans vorzunehmen. Die Steuereinheit 13 kann zudem wie in Figur 1 dargestellt optional mit der Warn- und/oder Stoppeinrichtung 11 sowie mit dem Zahnbehandlungs- oder -untersuchungsinstrument 1 verbunden sein. Dies ermöglicht es der Warn- und/oder Stoppeinrichtung 11, ein Stoppsignal an die Steuereinheit 13 auszugeben, wenn der Mindestabstand erreicht oder unterschritten ist. Die Steuereinheit 13 veranlasst dann das Anhalten des Zahnbehandlungs- oder -untersuchungsinstrumentes 1.

Ein Ausführungsbeispiel für das Zahnbehandlungs- oder -untersuchungsinstrument 1 aus Figur 1 ist in Figur 2 im Detail dargestellt. Wie bereits erwähnt ist es als zahnmedizinischer Bohrer ausgestaltet. Dieser umfasst den Handgriff 3, eine Bohrerkopf 6 und ein Bohrwerkzeug 37, das am Bohrerkopf 6 angeordnet ist und von einem nicht dargestellten Elektromotor angetrieben wird.

Ebenfalls in Figur 2 darstellt ist ein Zahn 39, bei dem die Pulpa 41, das Dentin 43 und der Zahnschmelz 45 zu erkennen sind. Das Bohrwerkzeug 37 findet dazu Verwendung, Zahnschmelz 45 und Dentin 39 aufzubohren, beispielsweise um Karies zu entfernen. Dabei soll das Bohrwerkzeug 37 die empfindliche Pulpa 41 nicht erreichen. Die mit Bezug auf Figur 1 beschriebene erfindungsgemäße Zahnbehandlungs- oder -untersuchungsvorrichtung gibt daher ein Warnsignal aus und/oder hält das Bohrwerkzeug 37 an, wenn der Abstand d zwischen der Oberfläche 47 der Bohrung und der Pulpa 41 einen Mindestabstand erreicht beziehungsweise unterschreitet.

Um die Messstrahlung dem Zahn 39 zuzuführen, ist die Lichtleitfaser 25 des Messstrahlzweiges 19 durch das Handstück und das Bohrwerkzeug 37 bis zum distalen Ende des Bohrwerkzeuges 37 geführt, welches gleichzeitig das distale Ende 5 des Zahnbehandlungs- oder -untersuchungsinstrumentes 1 darstellt. Das distale Ende 31 der optischen Faser 25 befindet sich im distalen Ende des Bohrwerkzeugs 37. Gegebenenfalls kann es gegenüber dem distalen Ende des Bohrwerkzeugs 37 auch etwas zurückversetzt sein, um Beanspruchungen des distalen Faserendes 31 während des Bohrprozesses zu verringern.

Die Lichtleitfaser 25 ist in der Rotationsachse des Bohrwerkzeuges 37 durch dieses geführt. Hierbei ist das Bohrwerkzeug entweder entlang seiner Rotationsachse derart hohl ausgestaltet, dass sich das Bohrwerkzeug 37 um die optische Faser 25 herum drehen kann, oder die optische Faser 25 umfasst ein distales Endstück, welches über einen drehbaren Koppler mit dem Rest der optischen Faser gekoppelt ist. In diesem Fall kann das distale Endstück der optischen Faser zusammen mit dem Bohrwerkzeug 37 rotieren.

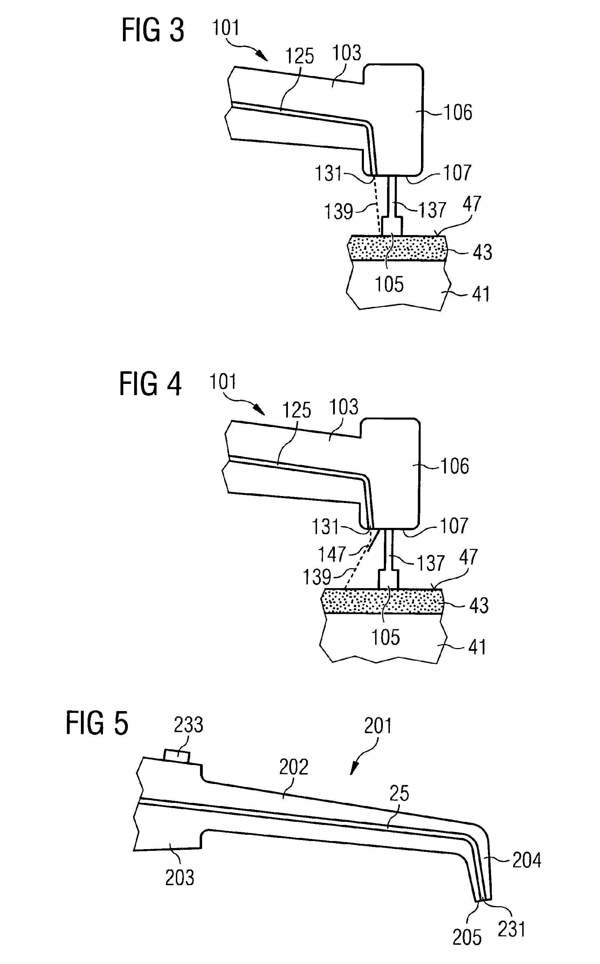

Eine alternative Ausführungsvariante des Bohrers aus Figur 2 ist in Figur 3 dargestellt. Die Figur zeigt außerdem einen Ausschnitt aus dem Zahn, in dem das Dentin 43 und die Pulpa 41 zu erkennen sind.

Wie der Bohrer 1 aus Figur 2 weist auch der Bohrer 101 aus Figur 3 ein Handstück 103 auf, an dessen Ende ein Bohrwerkzeug 137 angeordnet ist. Im Unterschied zum Bohrer 1 aus Figur 2 ist die optische Faser 125 in der vorliegenden Ausführungsvariante nicht durch das Bohrwerkzeug 137 geführt, sondern endet an der Unterseite des Bohrerkopfes 106. Die Anordnung des distalen Faserendes 131 im Bohrerkopf 106 ist so gewählt, dass austretende Messstrahlung am Ort des distalen Endes des Bohrwerkzeuges 137 auf die Oberfläche 47 trifft. Dies ist in Figur 3 durch die gestrichelte Linie 139 dargestellt. Die mit Bezug auf Figur 3 beschriebene Variante des Bohrers 101 weist gegenüber der mit Bezug auf Figur 2 beschriebenen Variante einen deutlich einfacheren mechanischen Aufbau auf. Außerdem können weiterhin Standardbohrwerkzeuge Verwendung finden.

Eine weitere alternative Ausführungsvariante des Bohrers ist in Figur 4 dargestellt. Diese Ausführungsvariante ähnelt stark des Ausführungsvariante aus Figur 3. Elemente, die mit Elementen des Bohrers 101 aus Figur 3 übereinstimmen, sind in Figur 4 daher mit denselben Bezugsziffern wie in Figur 3 bezeichnet und werden nicht noch einmal beschrieben, um Wiederholungen zu vermeiden.

Der in Figur 4 gezeigte Bohrer 101 unterscheidet sich von dem in Figur 3 gezeigten Bohrer lediglich dadurch, dass an der Unterseite 107 des Bohrerkopfes 106 eine Scanvorrichtung 147 vorhanden ist, mit der der Auftreffpunkt des Strahles 139 auf der Oberfläche 47 lateral versetzt werden kann. Die Scanvorrichtung 147 kann beispielsweise in Form eines oder mehrerer Galvanometerspiegel realisiert sein, die sich in ihrer Orientierung durch elektrische Signale einstellen lassen. Mithilfe der Scanvorrichtung 147 können Tiefenscans in einem lateralen Bereich der Oberfläche 47 durchgeführt werden, was tomografische Aufnahmen eines Zahnvolumens ermöglicht und daher auch die Möglichkeit bietet, den lateralen Verlauf der Grenzfläche zwischen der Pulpa 41 und dem Dentin 43 zu ermitteln.

Statt durch die beispielhaft genannten Galvanometerspiegel kann die Scanvorrichtung auch durch eine Verschiebeeinrichtung realisiert sein, mit der sich das distale Faserende 131 entlang der Unterseite 107 des Bohrerkopfes 106 in mindestens eine Richtung, vorzugsweise jedoch in zwei Richtungen verschieben lässt.

Ein alternatives Ausführungsbeispiel für das Zahnbehandlungs- oder untersuchungsinstrument aus Figur 1 ist in Figur 5 dargestellt. Das dargestellte Instrument ist lediglich ein Untersuchungsinstrument 201, dessen einzige Aufgabe darin besteht, den Abstand der Pulpa von der aktuellen Oberfläche, also der Oberfläche des Zahnes oder einer Bohrung, zu ermitteln. Das Untersuchungsinstrument 201 umfasst ein Handstück 203, das einen nadelförmigen Fortsatz 202 mit einem abgewickelten Abschnitt 204 aufweist. Die Form des Untersuchungsinstruments 201 entspricht daher weitgehend einer zahnmedizinischen Sonde.

Durch das Handstück 203 und den nadelförmigen Fortsatz 202 ist die Lichtleitfaser 25 des Messzweiges des Weißlichtinterferometers 7 aus Figur 1 bis zum distalen Ende 205 des nadelförmigen Fortsatzes 202 geführt. Die optische Faser 225 weist ein distales Faserende 231 auf, welches mit dem distalen Ende 205 des nadelförmigen Fortsatzes 202 übereinstimmt.

Zum Untersuchen des Zahnes wird das distale Ende 205 des Fortsatzes 202 mit dem darin angeordneten distalen Faserende 231 an diejenige Stelle des Zahnes geführt, an dem der Abstand der Pulpa von der Zahnoberfläche ermittelt werden soll. Das Handstück 203 weist zudem eine Auslöstaste 233 auf, die mit der Steuereinheit 13 verbunden ist und über die die Steuereinheit 13 veranlasst werden kann, ein Auslösesignal an das Weißlichtinterferometer 7 auszugeben, mit dem eine weißlichtinterferometrische Messung des Abstandes der Pulpa von der Oberfläche, auf die das distale Ende 205 aufgesetzt ist, ausgelöst wird. Wenn die Messung ergibt, dass der Mindestabstand erreicht oder unterschritten ist, wird ein optisches oder akustisches Warnsignal ausgegeben.

Die erfindungsgemäße Zahnbehandlungs- oder -untersuchungsvorrichtung ermöglicht es, mithilfe eines der erfindungsgemäßen Verfahren das unbeabsichtigte Öffnen der Pulpa zu vermeiden. Dabei kann entweder das Behandlungsinstrument angehalten werden, oder es kann ein Warnsignal ausgegeben werden, welches das Bedienpersonal dazu veranlassen soll, das Behandlungsinstrument abzuschalten.

## Patentansprüche

1. Zahnbehandlungs- oder -untersuchungsvorrichtung mit
- einem Weißlichtinterferometer (7), welches einen Messstrahlzweig (19), einen Referenzstrahlzweig (17), einen Emitter (31, 131, 231) zum Emittieren von Messstrahlung in Richtung auf einen zu behandelnden bzw. zu untersuchenden Zahn, einen Empfänger (31, 131, 231) zum Empfang von Messstrahlung, welche vom zu behandelnden bzw. zu untersuchenden Zahn reflektiert worden ist, einen Überlagerer (21) zum Überlagern von Referenzstrahlung und reflektierter Messstrahlung und eine Detektor (23) zum Detektieren der überlagerten Strahlung und zum Erzeugen eines die überlagerte Strahlung repräsentierenden Interferometriesignals umfasst,
- einem Zahnbehandlungs- oder -untersuchungsinstrument (1, 101, 201) mit einem distalen Ende (5, 105, 205), in dem der Emitter (31, 131, 231) und der Empfänger (31, 131, 231) angeordnet sind und das derart ausgestaltet ist, dass es an den zu behandelnden bzw. zu untersuchenden Zahn eines Patienten herangeführt werden kann,
- einer dem Weißlichtinterferometer (7) zugeordneten Steuereinheit (13) mit einem Steuerprogramm, welches das Weißlichtinterferometer (7) dazu veranlasst, Tiefenscans vom Behandlungs- bzw. Untersuchungsobjekt durchzuführen,
- einer mit dem Detektor (23) zum Empfang des Interferometriesignals verbundenen Analyseeinrichtung (9), die zum Ermitteln des Abstands der Pulpa des zu behandelnden bzw. zu untersuchenden Zahns von dessen aktueller Oberfläche und zur Ausgabe eines den Abstand repräsentierenden Abstandssignals ausgebildet ist, und
- einer mit der Analyseeinrichtung (9) zum Empfang des Abstands verbundenen Warn- und/oder Stoppeinrichtung (11), die zum Ausgeben eines Warnsignals und/oder zum Anhalten des Zahnbehandlungs- oder -untersuchungsinstruments (1, 101, 201), wenn ein vorbestimmter Mindestabstand erreicht oder unterschritten ist, ausgestaltet ist.

2. Zahnbehandlungs- oder -untersuchungsvorrichtung nach Anspruch 1, in der der Messstrahlzweig (19) eine optische Faser (25) mit einem im distalen Ende (5, 105, 205) des Zahnbehandlungs- oder -untersuchungsinstruments (1, 101, 201) angeordneten distalen Faserende (31, 131, 231) umfasst und der Emitter und der Empfänger durch das distale Faserende (1, 131, 231) gebildet sind.

3. Zahnbehandlungs- oder -untersuchungsvorrichtung nach Anspruch 2, in der das Zahnbehandlungsinstrument (1, 101) ein Bohrer mit einem Bohrwerkzeug (37, 137) mit einem distalen Bohrwerkzeugende (37, 137) ist und das distale Faserende (31, 131) im distalen Bohrwerkzeugende (5, 105) oder in dessen Nähe angeordnet ist.

4. Zahnbehandlungs- oder -untersuchungsvorrichtung nach Anspruch 3, in der die optische Faser (25) in der Rotationsachse des Bohrwerkzeuges (37) des Bohrers angeordnet ist.

5. Zahnbehandlungs- oder -untersuchungsvorrichtung nach Anspruch 3, in der die optische Faser (25) außerhalb des Bohrwerkzeuges (137) des Bohrers (101) so angeordnet ist, dass das distale Faserende (131) auf denjenigen Ort abgebildet wird, an dem sich das distale Bohrwerkzeugende (105) befindet.

6. Zahnbehandlungs- oder -untersuchungsvorrichtung nach einem der Ansprüche 1 bis 5, in der Steuereinheit (13) mit einem Steuerprogramm versehen ist, welches das Weißlichtinterferometer (7) dazu veranlasst, kontinuierlich Tiefenscans vom Behandlungs- bzw. Untersuchungsobjekt durchzuführen.

7. Zahnbehandlungs- oder -untersuchungsvorrichtung nach einem der Ansprüche 1 bis 5, die ein vom Behandlungs- bzw. Untersuchungspersonal zu bedienendes Auslöseelement (233) umfasst, welches mit der Steuereinheit (13) zur Abgabe eines einen Tiefenscan auslösenden Auslösesignals verbunden ist.

8. Zahnbehandlungs- oder -untersuchungsvorrichtung nach einem der Ansprüche 1 bis 7, **dadurch gekennzeichnet, dass** der Messstrahlzweig (19) eine Scanvorrichtung (147) umfasst, mit der der Messstrahl auf dem zu behandelnden bzw. zu untersuchenden Zahn lateral versetzt werden kann.

9. Verfahren zum Steuern eines Zahnbehandlungs- oder -untersuchungsinstruments (1, 101, 201), in dem während der Zahnbehandlung bzw. der Zahnuntersuchung mittels eines Weißlichtinterferometrieverfahrens ein Tiefenscan des behandelten bzw. untersuchten Zahnbereiches vorgenommen, der Abstand des behandelten bzw. untersuchten Zahnbereiches von der Pulpa des Zahnes aus dem Tiefenscan ermittelt wird und das Zahnbehandlungs- oder -untersuchungsinstrument (1, 101, 201) angehalten wird, wenn ein bestimmter Mindestabstand erreicht oder unterschritten wird.

10. Verfahren zum Überwachen eines Zahnbehandlungs- oder -untersuchungsinstruments (1, 101, 201), in dem während der Zahnbehandlung bzw. der Zahnuntersuchung mittels eines Weißlichtinterferometrieverfahrens ein Tiefenscan des behandelten bzw. untersuchten Zahnbereiches vorgenommen, der Abstand des behandelten bzw. untersuchten Zahnbereiches von der Pulpa des Zahnes aus dem Tiefenscan ermittelt wird und ein Warnsignal abgegeben wird, wenn ein bestimmter Mindestabstand erreicht oder unterschritten wird.

11. Verfahren nach Anspruch 9 oder Anspruch 10, in dem zusätzlich zum Tiefenscan ein laterales Scannen des behandelten bzw. untersuchten Zahnbereiches erfolgt.

12. Verfahren nach Anspruch 11, in dem an jedem lateralen Punkt des lateralen Scans ein Tiefenscan vorgenommen wird.

13. Verfahren nach einem der Ansprüche 9 bis 12, in dem kontinuierlich Tiefenscans vorgenommen werden.

14. Verfahren nach einem der Ansprüche 9 bis 12, in dem ein Tiefenscan nach Betätigen einer Auslösevorrichtung (233) durch das Behandlungs- bzw. Untersuchungspersonal vorgenommen wird.

15. Verfahren nach einem der Ansprüche 9 bis 14, in dem in einem Tiefenscan ein Tiefenbereich von 1 bis 3 mm vermessen wird.
